(19) **Europäisches Patentamt / European Patent Office / Office européen des brevets**

(11) **EP 3 331 441 B1**

(12) ## FASCICULE DE BREVET EUROPEEN

(45) Date de publication et mention de la délivrance du brevet:
**29.01.2025 Bulletin 2025/05**

(21) Numéro de dépôt: **16750779.7**

(22) Date de dépôt: **05.08.2016**

(51) Classification Internationale des Brevets (IPC):
**A61B 5/11** *(2006.01)* **G01C 22/00** *(2006.01)*

(52) Classification Coopérative des Brevets (CPC):
**A61B 5/112; A61B 5/6829; A61B 5/7246;**
A61B 5/6831; A61B 2562/0219; G01C 22/006

(86) Numéro de dépôt international:
**PCT/EP2016/068796**

(87) Numéro de publication internationale:
**WO 2017/021545 (09.02.2017 Gazette 2017/06)**

(54) **PROCÉDÉ DE CARACTÉRISATION DE DÉMARCHE**

VERFAHREN ZUR CHARAKTERISIERUNG EINES GANGES

METHOD FOR CHARACTERISING A GAIT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.08.2015 FR 1557582**
**20.04.2016 EP 16020149**

(43) Date de publication de la demande:
**13.06.2018 Bulletin 2018/24**

(73) Titulaires:
- **Université Paris Cité**
  **75006 Paris (FR)**
- **Centre National de la Recherche Scientifique (CNRS)**
  **75016 Paris (FR)**
- **Ecole Normale Supérieure Paris-Saclay**
  **91190 Gif-sur-Yvette (FR)**
- **Etat Français - Ministère De La Défense Direction Centrale du Service de Santé des Armées**
  **92141 Clamart Cedex (FR)**
- **Univ Paris XIII Paris-Nord Villetaneuse**
  **93430 Villetaneuse (FR)**

(72) Inventeurs:
- **DADASHI, Robert**
  **67000 Strasbourg (FR)**
- **MOREAU, Thomas**
  **74500 Saint Paul en Chablais (FR)**
- **TRUONG, Charles**
  **95460 Ezanville (FR)**
- **DE WAELE, Catherine**
  **75005 Paris (FR)**
- **YELNIK, Alain**
  **92240 Malakoff (FR)**
- **BARROIS-MULLER, Rémi**
  **75018 Paris (FR)**
- **VAYATIS, Nicolas**
  **75005 Paris (FR)**
- **OUDRE, Laurent**
  **75012 Paris (FR)**
- **VIDAL, Pierre Paul**
  **75005 Paris (FR)**
- **RICARD, Damien**
  **75005 Paris (FR)**

(74) Mandataire: **Regimbeau**
**20, rue de Chazelles**
**75847 Paris Cedex 17 (FR)**

(56) Documents cités:
**EP-A1- 2 439 492     US-A1- 2007 021 689**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

DOMAINE GENERAL

La présente invention concerne un procédé et un système pour caractériser la démarche d'une personne.

ETAT DE LA TECHNIQUE

Les procédés visant à caractériser la démarche d'une personne sont de deux types : les procédés de simple comptage de pas, et les procédés de détection de pas, plus complexes car visant à déterminer des instants caractéristiques du pas bien précis, tel que l'instant de pose au sol du talon.

La majorité des procédés de détection de pas connus est basée sur une succession d'étapes ad hoc de traitement appliquées à un signal de mouvement mesuré sur un sujet cible.

On connaît par exemple le prétraitement de Pan-Tomkins, décrit dans l'article « "A real-time QRS détection algorithm", par J. Pan et W. J Tompkins. Ce prétraitement comprend un filtrage passe bande, une dérivation, une mise au carré, une intégration et une recherche de pics appliqués au signal de mouvement mesuré sur le sujet cible.

Un inconvénient important de ces procédés est qu'ils requièrent la calibration de nombreux paramètres (tailles des filtres, valeurs des seuils, critères de sélection de pics, etc...) qui sont bien souvent difficiles à estimer ou à apprendre, et sont souvent choisis de façon empirique. De plus, ces procédés reposent sur un $\alpha$ *priori* très fort sur la forme que doit avoir un pas, ce qui limite clairement leur efficacité sur des sujets cibles dont le pas est de forme non conventionnelle, par exemple des personnes atteintes d'un trouble de la démarche. Les documents EP 2439492 A1 (HONEYWELL, 2012-04-11) et US 2007/021689 A1 (STERGIOU NIKOLAOS ET AL, 2007-01-25) sont pertinents pour la compréhension de l'état de la technique en rapport avec la présente invention.

RESUME DE L'INVENTION

Un but de l'invention est de caractériser la démarche d'un sujet sans pour autant requérir d'étape préliminaire de calibration. Cet objectif peut inclure le fait de détecter des pas ou de déterminer une information caractéristique de la démarche du sujet.

Un autre but de l'invention est de pouvoir mettre en oeuvre un procédé de caractérisation de démarche qui puisse fonctionner aussi bien sur des sujets atteints de troubles de la démarche que de sujets sains.

Il est donc proposé selon un premier aspect de l'invention un procédé de caractérisation de démarche suivant la revendication 1.

Le procédé de caractérisation de démarche ci-dessus proposé peut être complété à l'aide des caractéristiques suivantes prises seules ou bien en combinaison lorsque cela est techniquement possible.

Le procédé peut comprendre une étape d'identification, dans le signal de mesure, d'au moins un instant de repère caractéristique d'un pas du sujet cible, en fonction du signal de référence à partir duquel le maximum local d'intérêt sélectionné a été produit.

Le procédé peut comprendre une étape de comptage du nombre de maximum local d'intérêt sélectionné correspondant à un signal de référence donné.

Le procédé peut comprendre une étape de comptage du nombre de maximum local d'intérêt lié chaque classes de signaux de référence, chaque classes de signaux de référence ayant été générées à partir d'informations caractéristiques des sujets de référence.

Au moins un signal de référence peut être comparé avec plusieurs portions du signal de mesure temporellement décalées les unes des autres, de sorte à produire une séquence de coefficients de similarité ordonnée d'après les positions temporelles des portions du signal de mesure ; un coefficient de similarité d'une séquence est alors sélectionné comme maximum local en fonction du résultat d'une comparaison entre la valeur du coefficient avec la valeur du coefficient qui le précède dans la séquence, et du résultat d'une comparaison entre la valeur du coefficient avec la valeur du coefficient qui le suit dans la même séquence.

Chaque signal de référence comprenant au moins un instant de repère prédéterminé et caractéristique d'un pas d'un sujet de référence, au moins un signal de référence peut être comparé avec plusieurs portions du signal de mesure temporellement décalées les unes des autres, et l'instant de repère caractéristique d'un pas du sujet cible être identifié à partir de l'instant de repère prédéterminé du signal de référence à partir duquel a été produit le maximum local d'intérêt sélectionné.

Un maximum local peut ne pas être sélectionné lorsqu'il a une valeur inférieure à un seuil prédéterminé.

Les maximums locaux peuvent être sélectionnés par ordre décroissant de valeur des coefficients de similarités.

Ainsi que définit dans la revendication 1, un maximum local ne peut pas être sélectionné lorsque la portion du signal mesuré, à partir duquel il a été produit, chevauche temporellement une autre portion du signal mesuré à partir de laquelle a été produit un maximum local déjà sélectionné. Un tel chevauchement permet d'accélérer la recherche de maxima locaux et par conséquent réduit la durée de mise en oeuvre du procédé de caractérisation de démarche.

Le procédé peut comprendre le calcul d'un rapport entre des caractéristiques dimensionnelles des deux signaux comparés. Dans ce cas, l'étape d'identification n'est pas mise en oeuvre pour un maximum local produit à partir des signaux comparés dont le rapport de dimension calculé franchit un seuil prédéterminé.

Les caractéristiques dimensionnelles peuvent être des amplitudes ou des variances des signaux comparés.

L'étape de comparaison peut comprendre une corrélation produisant un coefficient linéaire de Pearson, le coefficient de similarité dépendant de la valeur absolue du coefficient linéaire de Pearson.

La pluralité de signaux de référence peut comprendre des signaux de référence de durées différentes.

Le signal de mesure peut être un signal représentatif du mouvement d'un pied du sujet cible.

Le signal de mesure peut être ou dépendre d'au moins des signaux suivants :

- un signal d'accélération du sujet cible suivant une direction normale au sol, et/ou
- un signal d'accélération du sujet cible suivant une direction normale à une surface supérieure d'un pied du sujet cible, et/ou
- un signal de vitesse angulaire autour d'un axe de rotation d'un pied de la cible.

Le procédé peut comprendre l'acquisition du signal de mesure par au moins un capteur de mouvement attaché au sujet cible.

Au moins un signal de référence peut être acquis au cours d'une séquence de marche comprenant une marche en ligne droite d'au moins 5 mètres d'un sujet de référence.

Selon un deuxième aspect, il est en outre proposé un système de caractérisation de démarche suivant la revendication 7.

Le système, selon ce deuxième aspect, peut comprendre en outre au moins un capteur de mouvement adapté pour fournir le signal de mesure à l'interface de réception.

Ce système peut en outre comprendre une mémoire dans laquelle est mémorisée la pluralité de signaux de référence.

## DESCRIPTION DES FIGURES

La figure 1 représente de façon schématique un système de caractérisation de démarche et le pied d'un sujet vu de profil.

La figure 2 est un organigramme d'étapes d'une phase préliminaire à un procédé de caractérisation de démarche, selon un mode de réalisation de l'invention.

Les figures 3a et 3b représentent deux signaux de mesure acquis par mise en oeuvre de la phase préliminaire de la figure 3.

La figure 4 est un organigramme d'étapes d'un procédé de caractérisation de démarche selon un mode de réalisation de l'invention.

La figure 5 illustre un signal de référence et un signal cible comparés au cours de la mise en oeuvre du procédé de détection de la figure 4.

Sur les figures, les éléments identiques sont désignés par des références identiques.

## DESCRIPTION DETAILLEE

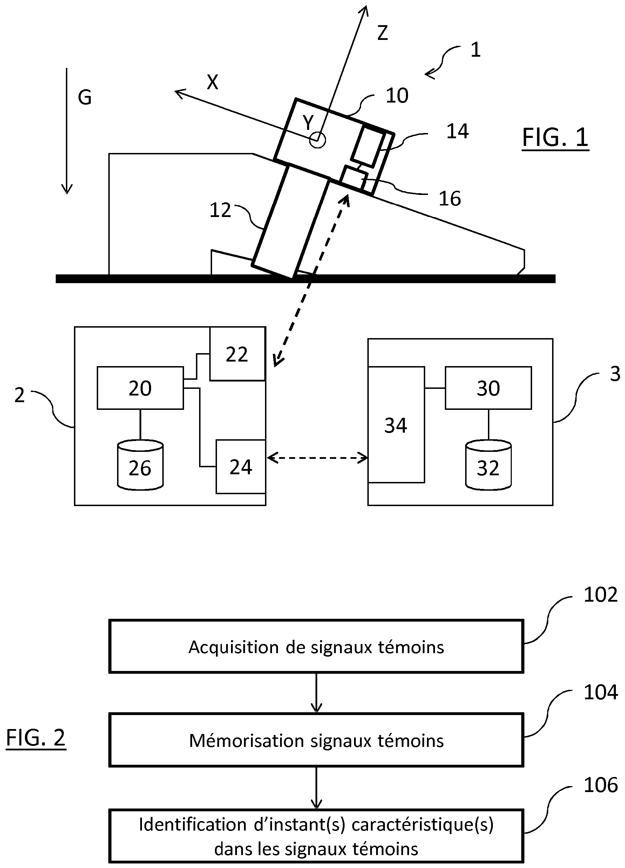

En référence à la **figure 1**, un système de caractérisation de démarche comprend un dispositif de mesure 1, un dispositif de traitement de signaux 2 et un serveur 3.

Le dispositif de mesure 1 comprend au moins un capteur de mouvement 10 et une ceinture 12.

La ceinture 12 est adaptée pour être attachée autour du pied d'un sujet, par exemple entre le talon et les doigts de pied comme représenté sur la figure 1. Les capteurs de mouvements 10 sont fixés à la ceinture 12 de sorte à ne pas gêner la marche du pied auquel la ceinture 12 est attachée.

Par exemple, il est prévu dans le dispositif de mesure 1 trois capteurs de mouvement 10, afin de mesurer des mouvements du pied suivant trois axes X, Y et Z définissant ensemble un repère de mesure attaché au dispositif 1.

On utilise, par exemple, au moins un capteur de vitesse angulaire autour d'un des axes précités et/ou au moins un capteur d'accélération le long d'un des axes précités.

Le dispositif de mesure peut également comprendre au moins un magnétomètre. Par exemple, il est prévu trois magnétomètres, chaque magnétomètre étant configuré pour acquérir une composante de champ magnétique suivant l'un des respectifs axes X, Y, Z.

Le dispositif de mesure 1 comprend par ailleurs des moyens de traitement de signal internes 14, et une interface de communication 16.

Les moyens 14 de traitement de signal sont configurés pour élaborer au moins un signal de mouvement de sortie à partir d'au moins un des signaux élémentaires fournis par chacun des différents capteurs de mouvement.

L'interface de communication 16 est configurée pour communiquer avec le dispositif de traitement de signaux 2. L'interface de communication est notamment apte à transmettre au dispositif de traitements de signaux 2 chaque signal de sortie produit par les moyens de traitements internes du dispositif de mesure 1.

L'interface de communication 16 est de préférence sans fil pour ne pas gêner la marche du sujet lorsque le dispositif de mesure 1 est attaché à son pied.

Dans un mode de réalisation, le dispositif de mesure 1 est un dispositif commercialisé par la société Xsens, lequel est fixé à un pied grâce à une bande à velours-crochet, et les signaux de sortie sont échantillonnés à 100 Hz.

Le dispositif 2 de traitements de signaux comprend au moins un processeur 20, une première interface de communications 22, une deuxième interface de communication 24 et une mémoire interne 26.

Le processeur 20 est configuré pour mettre en oeuvre un procédé de caractérisation de démarche codé sous la forme d'instructions de code de programme, sur la base de signaux reçus via les deux interfaces de communications 22 et 24.

L'interface de communication 22 est adaptée pour recevoir les signaux de sortie émis par le dispositif de mesure 1, par exemple au moyen du protocole Wi-Fi ou du protocole Bluetooth.

L'interface de communication 24 est adaptée pour communiquer avec le serveur distant 3, via un réseau public tel qu'Internet.

Le dispositif 2 de traitements de signaux peut par exemple se présenter sous la forme d'un terminal mobile, d'un ordinateur portable, ou d'un PC.

Le serveur 3 comprend une interface de communication 34 pour communiquer avec le dispositif de traitements de signaux 2, un processeur 30 et une mémoire 32 adaptée pour mémoriser un ensemble de signaux de mesures, et des données attenantes à ces signaux, sous la forme d'une base de données.

On va à présent détailler les étapes d'un procédé de caractérisation de démarche mis en oeuvre au moyen du système de la figure 1.

*Phase préliminaire de collecte de signaux de référence*

En référence à la **figure 2**, une phase préliminaire de collecte de signaux de référence est mise en oeuvre.

Dans une première étape 102 de cette phase préliminaire, chaque signal de référence est mesuré sur un sujet de référence.

L'acquisition 102 d'un signal est mise en oeuvre au moyen du système d'acquisition illustré sur les figures 1 et 2. Pour ce faire, on attache un dispositif de mesure 1 à un pied d'un sujet de référence (voir deux dispositifs 1, l'un pour le pied gauche et l'autre pour le pied droit). Le sujet de référence est ensuite invité à marcher selon un protocole clinique prédéterminé.

Au cours de la marche du sujet de référence, les capteurs de mouvements 10 acquièrent des signaux représentatifs d'un paramètre de mouvement (accélération et/ou vitesse, par exemple vitesse angulaire) du pied selon les axes X, Y, Z, qui sont ensuite traités par les moyens de traitements internes du dispositif de mesure 1 pour générer au moins un signal d'accélération de référence du pied correspondant du sujet de référence.

Les moyens de traitement internes peuvent également utiliser les mesures de champ magnétiques acquises par les magnétomètres, si présents dans les dispositifs 1 de mesure.

Chacun des deux dispositifs de mesure 1 (pieds gauche et droit) acquiert neuf signaux bruts (accélérations 3D, vitesses angulaires 3D et champs magnétiques 3D) dans le repère lié au capteur défini par les axes X, Y et Z, ces signaux pouvant éventuellement être recalibrés ou calibrés dans un repère fixe par rapport au sol via les moyens 14 de traitements internes des dispositifs 1 de mesure, et ce afin d'obtenir les signaux de référence.

De préférence, on utilise comme signaux de référence certaines des composantes pertinentes pour caractériser une démarche. Trois composantes pertinentes pour caractériser la démarche, et représentées sur la figure 1, sont:

- un signal d'accélération du sujet de référence mesuré suivant une direction G normale au sol,
- un signal d'accélération du sujet de référence mesuré suivant l'axe Z lié au dispositif 1, lequel axe est normal à la surface supérieure d'un pied du sujet de référence,
- un signal de vitesse angulaire de rotation d'un pied du sujet de référence autour de l'axe Y au cours d'une marche en ligne droite du sujet de référence (cet axe Y étant perpendiculaire à l'axe Z et parallèle à un axe d'inclinaison du pied au niveau de la cheville).

Chaque signal de référence est ensuite transmis au dispositif de traitement 2, via l'interface 22, puis au serveur 3 via les interfaces 24 et 30.

Dans une étape 104, le signal de référence mesuré est mémorisé dans la mémoire 32 du serveur 3. Un signal de référence peut être mémorisé dans la mémoire interne 26 du dispositif de traitement 2, à la place de ou en complément de la mémoire 32 du serveur distant 3.

Dans une étape 106, chaque signal de référence mémorisé fait l'objet d'une annotation, visant à déterminer au moins un instant de repère caractéristique du pas du sujet de référence.

Cette étape d'annotation peut être mise en oeuvre de façon empirique par un praticien qui scrute la démarche du sujet de référence pendant l'acquisition 102 du signal de référence.

Par ailleurs, peut être associé à chaque signal de référence au moins une information caractéristique du sujet de référence correspondant, préalablement déterminée.

Une information caractéristique de démarche peut par exemple identifier une classe particulière de sujet, parmi plusieurs classes prédéterminées.

Une classe peut ainsi se rapporter à un type de démarche (normale, boitement, ...), une sévérité de l'atteinte à la démarche, une vitesse de démarche, l'âge du sujet de référence (jeune, âgé, ...), etc.

Par exemple, cette information peut se rapporter à une plainte exprimée par des sujets (sensoriel, moteur, équilibre, troubles cognitifs ou de l'ouïe, vertige, étourdissement ou douleur), à un déficit constaté et caractérisé (latéralisation, occurence chronique ou aiguë) du sujet (proprioceptif, vestibulaire, visuel, moteur : hémiparésie, paraparésie, syndrome parkinsonien...). Cette information peut inclure le niveau anatomique de la lésion : système nerveux central ou périphérique ou dans les organes sensoriels (vestibule central, vestibule périphérique, les nerfs périphériques, la moelle épinière, le tronc, le cervelet, articulations des membres inférieurs, Hémisphère cérébral: cortex, ganglions de la base ou de la matière blanche). Ces informations peuvent également porter sur l'étiologie de l'atteinte (vasculaire, inflammation, traumatisme, infection, tumeur avant ou après la chirurgie, dégénérative, toxique, métabolique, psychosomatique...).

Les mêmes étapes 102, 104, 106 sont mises en oeuvre de façon répétées pour plusieurs sujets de référence.

Au terme de la phase préliminaire, sont ainsi mémorisées dans la mémoire 32 une pluralité de signaux de référence, et chaque signal de référence est annoté avec au moins un instant caractéristique du pas du sujet de référence sur lequel le signal de référence a été mesuré, et/ou au moins une information caractéristique du sujet de référence.

Il est possible de mémoriser un signal dont la durée couvre au moins une partie d'un pas ou plusieurs pas du sujet de référence correspondant.

Néanmoins, il est préférable de mémoriser un signal de référence présentant une durée qui couvre au moins une partie d'un seul et unique pas du sujet de référence, de sorte à limiter la consommation de mémoire par le signal de référence, et d'éviter une redondance potentielle d'informations. Le ou chaque instant caractéristique mémorisé en sus du signal de référence lui-même se rapport(ent) à cet unique pas.

De plus, l'utilisation d'un seul et unique pas du sujet de référence ou d'une partie de ce pas permet d'augmenter la sensibilité et la spécificité de cette méthode. En outre elle permet de meilleurs résultats lorsque le pas du sujet cible est de forme non conventionnelle.

L'utilisation d'au moins une partie d'un seul et unique pas du sujet de référence correspond par exemple à l'utilisation d'une partie ou de plusieurs parties des phases du pas telle que la phase d'appui, le contact initial, le balancement...

En pratique, cette annotation est matérialisée par la mémorisation de données permettant de localiser les instants caractéristiques dans le signal de référence correspondant, ces données étant liées de façon logique avec ce signal de référence dans la mémoire 32 ou 26.

Les sujets de référence auprès desquels les signaux d'accélération de référence sont mesurés présentent de préférence des profils de démarche différents. De manière générale, les sujets de référence auprès desquels les signaux d'accélération de référence sont mesurés peuvent être des sujets sains ou des patients (sujet atteint d'une affection médicale).

Par exemple,

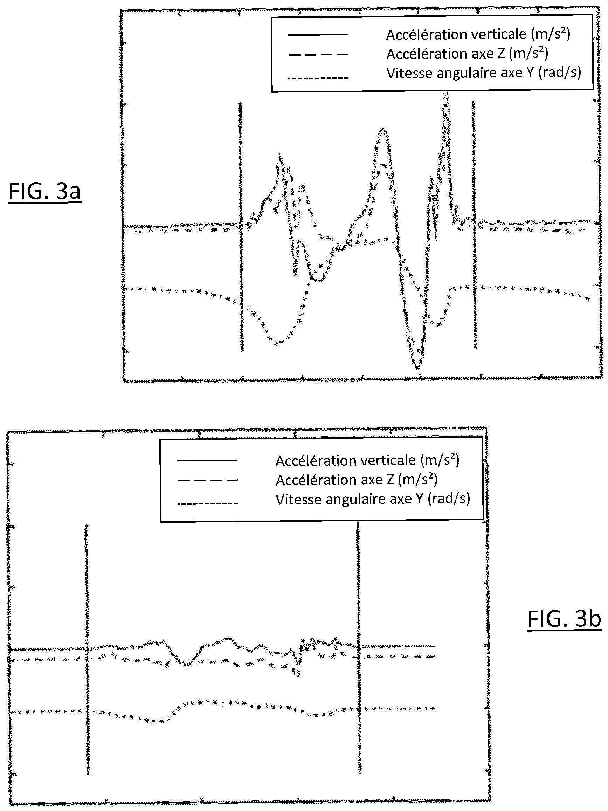

- au moins un des signaux de référence est mesuré sur un sujet atteint de trouble de la démarche (trois exemples de signaux de référence mesurés sur un patient atteint d'un problème à la hanche sont illustrés en **figure 3b**) ; et
- au moins un des signaux de référence est mesuré sur un sujet sain, c'est-à-dire un sujet qui n'est pas atteint de troubles de la démarche (tel que les trois signaux de référence représentés en **figure 3a**).

Ainsi, l'invention porte également sur un procédé de collecte d'informations sur la démarche telles que le style de marche ou une pathologie éventuelle. Ainsi, il est par exemple possible de distinguer de légères boiteries, des façons particulières de lever le pied, des jambes arquées, un chaloupement durant la marche. Même si une structure commune peut éventuellement être décelée entre les signaux de référence représentés sur les figures 3a et 3b, il convient de constater que leurs amplitudes sont très différentes (sur la figure 3b, le sujet a en effet tendance à traîner des pieds, ce qui modifie la structure et l'amplitude du pas comparativement au sujet de référence de la figure 3a).

Par ailleurs, les différents signaux de référence collectés ne sont pas forcément de même durée, ni associés à un nombre identique d'instant caractéristiques de pas. On comprend en effet, que, d'un sujet de référence à l'autre, un pas peut être de durée différente.

Les signaux de référence collectés constituent ainsi une bibliothèque de pas couvrant un large panel de démarches, que l'on va utiliser pour caractériser le pas d'un sujet qualifié dans la suite de sujet « cible », ce sujet cible étant de préférence différent des sujets de référence.

*Phase de caractérisation de démarche du sujet cible*

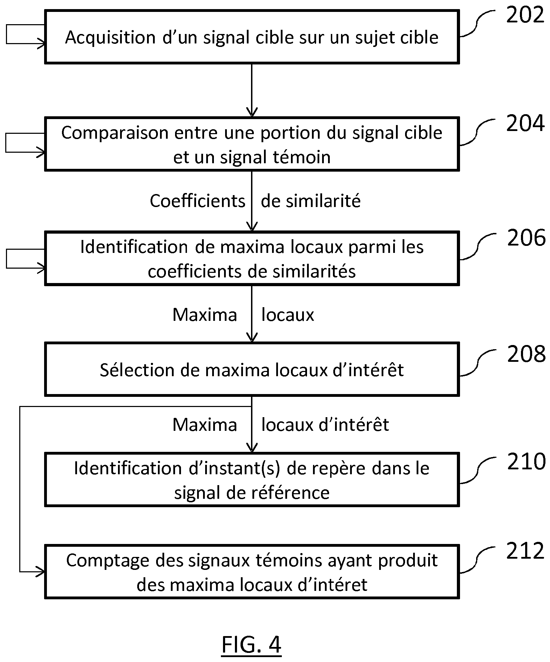

En référence à la **figure 4**, un procédé de caractérisation de démarche du sujet cible comprend les étapes suivantes.

Dans une étape 202, le dispositif de mesure 1, préalablement fixé à un pied du sujet cible, acquiert au moins un signal de mouvement du sujet cible, ce signal étant qualifié dans la suite de « signal cible ».

Le procédé selon l'invention peut être mis en oeuvre sur un signal cible ayant été préalablement mesuré.

Un signal cible est par exemple un signal d'accélération du pied le long de l'axe X, Y et/ou Z, ou un signal de vitesse angulaire du pied autour de l'axe X, Y et/ou Z.

Le signal cible et les signaux de référence sont donc mesurés sur des sujets différents. Afin d'améliorer la détection, le protocole d'acquisition des signaux de référence est représentatif des conditions dans lesquels le signal cible est généré.

Le signal cible est ensuite transmis au dispositif de traitement 2.

Dans une étape 204, une comparaison entre le signal cible et un signal de référence est mise en oeuvre.

Cette comparaison 204 peut être mise en oeuvre par le processeur 20 du dispositif de traitement 1 ; dans ce cas, le dispositif de traitement 1 envoie une requête au serveur 3 visant à télécharger un signal de référence en vue de cette comparaison 204. En variante, la comparaison 204 est mise en oeuvre par le serveur 3, le dispositif de traitement servant alors uniquement de relai de transmission du signal cible entre le dispositif de mesure 1 et le serveur 3.

La comparaison 204 est plus précisément mise en oeuvre pour différentes portions temporelles du signal cible décalées temporellement les unes par rapport aux autres dans le signal cible. On comprend alors que sont mises en oeuvre autant de comparaisons que de portions temporelles définies dans le signal cible.

Chaque portion temporelle du signal cible est de même durée T que le signal de référence auquel cette portion va être comparée.

Les différentes portions du signal cible peuvent être décalées les unes des autres d'un pas temporel constant.

Les différentes portions du signal cible peuvent se chevaucher.

La comparaison 204 d'une portion temporelle donnée du signal cible avec un signal de référence produit un coefficient représentatif d'une probabilité de similarité entre les deux signaux comparés, ce coefficient étant appelé dans la suite « coefficient de similarité ».

La comparaison 204 comprend par exemple une corrélation de la portion temporelle du signal cible avec le signal de référence ; dans ce cas, le coefficient de similarité dépend du coefficient linéaire de Pearson entre les deux signaux corrélés.

Le coefficient de similarité $\rho(x,y)$ entre deux signaux x et y peut plus précisément être exprimé comme la valeur absolue du coefficient linéaire de Pearson, comme suit :

$$\rho(x, y) = \frac{|cov(x, y)|}{std(x)\,std(y)}$$

où cov(x, y) désigne la covariance ces signaux x et y, *std*(*x*) et *std*(*y*) désignent les écarts-type respectifs des signaux x et y.

Dans ce cas, une valeur élevée de coefficient de similarité indiquera une forte similitude entre la portion temporelle du signal cible et le signal de référence comparés. En revanche, une valeur faible de ce coefficient de similarité indiquera une faible similitude entre les deux signaux comparés.

Pour N portions temporelles du signal cible, sont donc produits N coefficients de similarités ayant chacun une valeur spécifique, via N comparaisons 204. Les N comparaisons opérées balayent l'intégralité de la durée du signal cible.

L'ensemble des N coefficients de similarité produits à partir du signal cible et d'un même signal de référence donné est dans la suite appelé séquence de coefficients.

Les coefficients d'une séquence sont classés par ordre de position temporelle des N différentes portions temporelles du signal cible considérées lors de l'étape de comparaison 204. Un sens de parcours d'une séquence de coefficients correspond donc à un sens de parcours temporel du signal cible.

L'étape de comparaison 204 est répétée pour chaque signal de référence préalablement mémorisé lors de la phase préliminaire. On notera que, chaque signal de référence étant de durée T potentiellement distincte de celle des autres, les portions temporelles du signal cible qui lui sont comparées peuvent être également redéfinies différemment pour chaque signal de référence.

En croisant chaque signal de référence avec le signal cible, le nombre total de séquences de coefficients de similarité produit à l'issue de ces répétitions est égal au nombre de signaux cibles acquis simultanément sur le sujet cible, multiplié par le nombre de signaux de référence mémorisés.

Chaque séquence peut avoir un nombre de coefficients différent, en fonction du pas choisi pour décaler les portions temporelles les unes par rapport aux autres dans le signal cible correspondant, et en fonction de la longueur (durée) du signal de référence considéré.

Dans une étape 206, le processeur met en oeuvre une identification de maxima locaux dans une séquence donnée de coefficients de similarités.

Dans une séquence donnée, un coefficient courant constitue un maximum local si le coefficient suivant dans la séquence et le coefficient précédent dans la séquence ont des valeurs qui sont inférieures à celle du coefficient courant. La détection de maxima locaux procède typiquement par une série de comparaisons entre valeurs de coefficients adjacents dans une séquence.

Chaque maximum local est alors un coefficient de similarité produit à partir d'une portion du signal qui ressemble davantage au signal de référence que les portions adjacentes du signal cible.

Une séquence donnée peut ainsi comprendre zéro, un ou plusieurs maxima locaux.

L'étape 206 est répétée pour chaque séquence de coefficients produite.

Dans une étape de sélection 208, le processeur sélectionne, parmi plusieurs maxima locaux identifiés, certains maxima locaux d'intérêt.

Les maxima locaux d'intérêt sont sélectionnés sur la base de la valeur des coefficients de similarité : un maximum local n'est pas sélectionné comme maximum local d'intérêt si sa valeur est inférieure à un seuil prédéterminé $\lambda$, de sorte à éliminer des maxima locaux ayant des valeurs de probabilité de similarité faibles.

Par exemple, dans le cas de coefficients de similarités définis comme la valeur absolue de coefficients linéaires de Pearson, seuls les maxima locaux ayant une valeur supérieure ou égale à 0,5 sont retenus comme maxima locaux d'intérêt.

De préférence, le seuil prédéterminé $\lambda$ est compris entre 0,6 et 0,8.

La sélection 208 peut être avantageusement être mise en oeuvre par ordre décroissant de valeur des coefficients de similarité ; de la sorte, le nombre de comparaisons avec le seuil $\lambda$ est réduit.

Un autre critère de sélection 208 d'un maximum local est le chevauchement de la portion du signal cible correspondant avec la portion temporelle du même signal cible à partir duquel a été produit un autre coefficient de similarité, constituant non seulement un autre maximum local, et en plus ayant déjà été sélectionné comme maximum local d'intérêt parmi les maxima locaux de la séquence.

Autrement dit, sachant qu'un premier maximum local est produit à partir d'une comparaison entre une première portion du signal cible et un signal de référence, puis sélectionné comme maximum local d'intérêt,

- un deuxième maximum local produit à partir d'une comparaison entre le signal de référence et une deuxième portion qui chevauche la première portion n'est pas sélectionné comme maximum local d'intérêt, et
- un troisième maximum local produit à partir d'une comparaison entre le signal de référence et une troisième portion qui ne chevauche pas la première portion est bien sélectionné comme maximum local d'intérêt.

De préférence, l'étape de sélection 208 des maxima locaux d'intérêt est mise en oeuvre par ordre de valeur décroissante de valeur de coefficient ; on est de la sorte certain que les maxima locaux ayant les plus grandes valeurs dans la séquence seront sélectionnées comme maximum local d'intérêt.

Dans la mesure où autant de séquences que de signaux de référence peuvent être produites, l'ordre de sélection 208 peut se faire après un tri de l'ensemble des maxima locaux répertoriés dans l'ensemble des séquences.

A l'issue de l'étape de sélection 208, on dispose d'au moins un maximum local d'intérêt, lequel est associé à une portion spécifique du signal cible ; et quand une pluralité de maxima locaux d'intérêts sont produits, il est certain que les portions spécifiques du signal cible associées sont temporellement disjointes dans le signal cible (c'est-à-dire ne se chevauchent pas).

Dans une étape d'identification 210, le processeur identifie dans le signal cible au moins un instant caractéristique d'un pas du sujet cible sur la base du ou des maxima locaux d'intérêt sélectionnés.

Cette étape d'identification 210 comprend le téléchargement depuis le serveur 3 vers le dispositif de traitement 2, du ou des instant(s) de repère caractéristique(s) annotés dans le signal de référence ici considéré, lors de la phase préliminaire de collecte des signaux de référence. En variante, l'étape d'identification est mise en oeuvre par le serveur 3.

Un instant de repère caractéristique du sujet cible est identifié dans le signal cible en examinant un maximum local d'intérêt sélectionné dans l'étape 208.

Pour rappel, le signal de référence ayant conduit à l'obtention d'un maximum local d'intérêt donné a été annoté avec au moins un instant caractéristique au cours de la phase préliminaire et/ou avec au moins une caractéristique du sujet de référence correspondant.

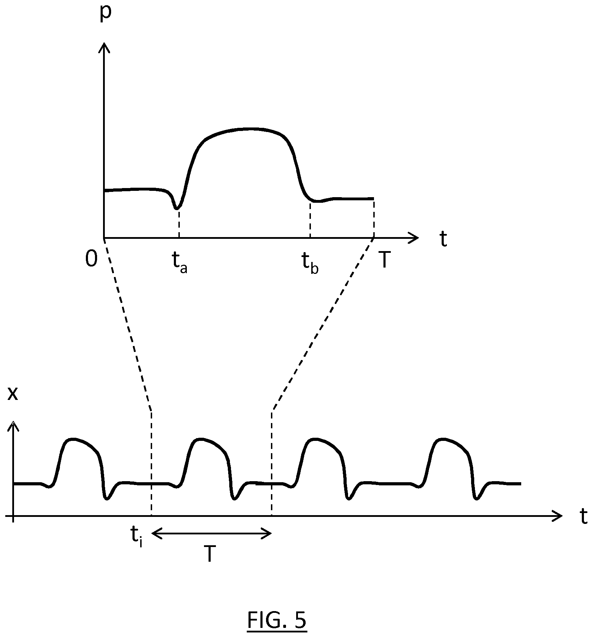

On a par exemple représenté en **figure 5** un signal de référence p comprenant deux instants caractéristiques : un instant de début de pas $t_a$ et un instant de fin de pas $t_b$.

L'instant caractéristique $t_a$ est reporté dans la portion temporelle du signal cible à la position temporelle $t_i + t_a$, où $t_i$ désigne l'instant de début de la portion temporelle dans le signal cible.

Similairement, l'instant caractéristique $t_b$ est reporté dans la portion temporelle du signal cible à la position temporelle $t_i + t_a$, où $t_i$ désigne l'instant de début de la portion temporelle dans le signal cible.

Chaque instant de repère prédéterminé au cours de la phase préliminaire est ainsi calqué dans le signal cible.

Le processeur 20 procède de la même manière pour tout autre maximum local d'intérêt sélectionné.

L'étape d'indentification 210 permet ainsi dans une application particulière de compter les pas du sujet cible.

Dans un mode de réalisation, l'identification 210 d'instant(s) de repère caractéristique(s) n'est pas forcément mise en oeuvre pour tous les maxima locaux sélectionnés au cours de l'étape de sélection 208. Pour chaque maximum local d'intérêt, le processeur calcule dans ce cas un rapport entre des caractéristiques dimensionnelles des deux signaux comparés sur la base desquels le maxima a été identifié 206, puis sélectionné 208.

Si ce rapport de dimension franchit un seuil prédéterminé μ, alors l'étape d'identification 210 d'instant caractéristique n'est pas faite dans la portion temporelle du signal cible.

Par exemple, si le numérateur du rapport est la caractéristique dimensionnelle se rapportant au signal cible et si le dénominateur du rapport est la caractéristique dimensionnelle se rapportant à un signal de référence, alors on vérifie si le rapport est inférieur au seuil μ, et si tel est le cas, alors l'étape d'identification 210 d'instant caractéristique n'est pas faite dans la portion temporelle du signal cible.

Sinon, l'étape d'identification 210 selon les modalités qui précèdent est mise en oeuvre sur cette portion temporelle du signal cible.

Le rapport de dimension peut être par exemple un rapport d'amplitude entre les signaux comparés ou un rapport de variance entre les signaux comparés.

Plusieurs rapports peuvent être calculés pour une paire de signaux comparés ; il peut alors être prévu de ne pas déclencher l'étape d'identification d'instant de repère si au moins un des rapports est supérieur à un seuil prédéfini.

De préférence, le calcul des rapports de dimension est mis en oeuvre après l'identification 206 des maxima locaux, très préférentiellement après la sélection des maxima locaux d'intérêt, de sorte à limiter le nombre de rapports à calculer.

Les étapes 202, 204, 206, 208 et 210 peuvent être répétées pour plusieurs signaux cibles acquis simultanément via des capteurs différents du dispositif de mesure 1 (par exemple des signaux d'accélération suivant des axes différents parmi ceux évoqués plus haut).

D'autres traitements que l'étape d'identification 210 peuvent être mis en oeuvre une fois que des maxima locaux ont été sélectionnés dans l'étape 208 de sélection.

Le processeur 20 peut par exemple compter 212 le nombre de fois qu'un signal de référence a permis de générer un maximum local d'intérêt de référence.

Le processeur 20 télécharge ensuite les informations caractéristiques des sujets de référence (recensées au cours de la phase préliminaire) qui sont associés aux signaux de référence ayant générés au moins un des maxima locaux d'intérêt.

Le processeur 20 peut par exemple calculer un pourcentage de représentation de chaque information caractéristique dans l'ensemble des maxima locaux d'intérêt et donc du signal cible.

Il est tout à faire possible qu'un signal de référence (ou plusieurs signaux de référence porteur d'une même information caractéristique de démarche), soit représenté de façon majoritaire dans les maxima locaux d'intérêts.

L'information caractéristique majoritaire peut ainsi être attribuée au sujet cible.

Le processeur 20 peut aussi, pour chaque classe de signaux de référence générées à partir d'informations caractéristiques des sujets de référence, compter 212 le nombre de maximum local d'intérêt lié à cette classe et donc ayant été associé au signal cible.

Le processeur 20 peut ensuite par exemple calculer un pourcentage de représentation de chaque information caractéristique dans le signal cible.

L'information caractéristique majoritaire peut ainsi être attribuée au sujet cible.

*Exemple d'implémentation algorithmique du procédé de détection*

Le procédé de détection peut être implémenté sous la forme d'un programme d'ordinateur comprenant des instructions de codes aptes à être exécutées par le processeur 20.

Il va être détaillé ci-après un algorithme consistant en un mode de réalisation possible de ce programme d'ordinateur.

On considère dans la suite que le dispositif de mesure 1 fournit un k-uplet de de signaux discrets mesurés sur un même sujet. Ce k-uplet peut être représenté par un vecteur de longueur |p| et de dimension k.

On introduit les notations suivantes :

- x est un vecteur représentatif d'un k-uplet de signaux cibles discrets mesurés le sujet cible.
- p est un vecteur représentatif d'un k-uplet de signaux de référence discrets mesurés sur un même sujet de référence.
- |P| est le nombre de vecteurs p.
- |x| (respectivement |p|) est la longueur du vecteur x (respectivement p), comptée en nombre d'échantillons.
- $x^{(k)}$ (respectivement $p^{(k)}$) est la k$^{ième}$ composante du vecteur x
- $\mathrm{x}_{i:j}^{(k)}$ est la portion temporelle de x(k) comprise entre les échantillons d'indices i et j ; on a donc $\mathrm{x}_{1:|\mathrm{x}|}^{(k)} = x^{(k)}$.

Les coefficients de similarités, notés *r*, sont calculés entre les signaux de référence et le signal cible au cours de l'étape de comparaison 204, pour toutes les portions temporelles possibles de longueur |p|, ce que fait un total de $3 \times |P| \times |x|$ coefficients de similarité :

$$\forall k, \forall p \in P, \forall 1 \leq x \leq |x|$$

$$r(k, p, i) = \rho\left(p^{(k)}, \mathrm{x}_{i:i+|\mathrm{p}|-1}^{(k)}\right)$$

où r(k, p, i) est le coefficient de similarité issu de la comparaison entre la k$^{ième}$ composante du signal de référence p et la k$^{ième}$ composante du signal cible à l'instant i, et où $\rho$ est un coefficient linéaire de Pearson en valeur absolue.

Au cours de l'étape 206, un coefficient *r(k,p,i)* est désigné comme étant un maximum local s'il est plus grand que ses voisins temporaux directs. L'ensemble L des maxima locaux est ainsi l'ensemble des coefficients de similarité *r(k,p,i)* vérifiant cumulativement les deux conditions suivantes:

$$r(k, p, i) > r(k, p, i - 1)$$

$$r(k, p, i) > r(k, p, i + 1)$$

L'ensemble L contient toutes les positions acceptable pour des pas du sujet cible ; chaque coefficient *r(k, p, i)* peut être interprété comme la vraisemblance de trouver, dans le signal cible à l'instant i, un pas similaire à celui du signal de référence d'indice p.

L'étape de sélection 208 est codée à la façon d'un processus glouton. A chaque itération de l'algorithme, on choisit la plus grande valeur *r(k\*, p\*,i\*)* de coefficient dans l'ensemble L :

- Si le pas *p\** positionné à l'instant *i\** chevauche un pas qui a déjà été détecté, on le rejette et on recommence le processus ;
- Si le pas *p\** peut être positionné à l'instant *i\** sans chevaucher les détections précédentes, il est considéré comme détecté et les indices compris entre *i\** et *i\* + |p\*|* - 1 sont interdits dans les itérations prochaines.

L'algorithme glouton de l'étape 208 de sélection prend fin lorsque tous les indices sont interdits, ou lorsque tous les maxima locaux de l'ensemble L ont été considérés, ou lorsque les valeurs des coefficients restant dans l'ensemble L sont toutes plus petites que le seuil λ prédéterminé. Ce seuil λ agit comme un paramètre d'accélération de l'algorithme ; on évite en effet de faire tourner l'algorithme trop longtemps.

L'algorithme glouton de l'étape 208 produit un ensemble S de maxima locaux d'intérêts.

L'étape d'identification 210 des instants caractéristiques est ensuite mise en oeuvre sur la base de cet ensemble 5 et du seuil μ. Comme indiqué plus haut, le seuil μ est utilisé pour rejeter certains instants caractéristiques, en cas d'écart dimensionnel trop grand entre une portion du signal cible et une portion de signal de référence associé à un maximum local d'intérêt inclus dans l'ensemble S.

Par exemple, le seuil prédéterminé μ est inférieur ou égale à 0,2 et de préférence est compris entre 0,05 et 0,2, de façon plus préférée entre 0,1 et 0,2.

*Résultats*

Les inventeurs ont pu obtenir des résultats particulièrement satisfaisants après l'établissement d'une base de données de signaux contenant 295 signaux mesurés auprès de 74 sujets dont 23 sains et 51 atteints de troubles orthopédiques divers au genou, à la hanche ou à la cheville, certains des sujets ayant réalisé le protocole plusieurs fois.

Le protocole clinique d'acquisition des signaux de référence, auquel ont été soumis les sujets de référence portant le dispositif de mesure 1 est le suivant :

- rester immobile pendant 6 secondes,
- marcher tout droit pendant 10 mètres à une vitesse de confort,
- faire demi-tour,
- revenir au point de départ,
- rester immobile pendant 2 secondes.

Tous les signaux de la base de données ont été annotés manuellement avec deux instants de repère caractéristiques :

- un instant de début de pas $t_a$, auquel le talon décolle du sol,
- un instant de fin de pas $t_b$, auquel les orteils sont posés sur le sol et stabilisés.

Au total, ont été mémorisés 9357 pas (4674 sur le pied droit et 4683 sur le pied gauche).

Le fonctionnement du procédé de détection a été testé au moyen de la méthode d'évaluation qui suit.

- On a choisi aléatoirement |P| pas dans la base de données qui constituent une base d'apprentissage.
- On a choisi également des pas dans la base de données constituant une base de test. Afin d'éviter le sur-apprentissage, tous les signaux mesurés sur des sujets présents dans la base d'apprentissage sont écartés de la base de test.
- On a appliqué le procédé de caractérisation de démarche à chaque signal de la base de test en utilisant les |P| signaux de la base d'apprentissage comme signaux de référence.
- On a ensuite calculé un score de précision et un score de rappel des instants caractéristiques de début et de fin de pas identifiés dans chaque signal de la base de test par application du procédé selon l'invention, en considérant qu'un pas est correct si la moyenne des instants détectés de début et de fin de pas tombe à un instant où un pas est effectivement présent au vu des annotations collectées par les praticiens au cours de la phase préliminaire de collecte décrite plus haut.
- On a moyenné ensuite les scores de précision et de rappel pour tous les signaux de la base de test.

Dans les résultats présentés dans la suite, ce procédé a été répété 100 fois et on s'est intéressé à la moyenne et à l'écart type des scores de précision et de rappel.

Le tableau 1 ci-dessous présente les précisions et rappels (moyenne, et écart-type entre parenthèses sur 100 simulations) pour différentes nombre de |P| de signaux de référence.

Tableau 1

| Valeur de |P| | Précision | Rappel |
|---|---|---|
| 5 | 96.6 (5.04) | 96.6 (1.04) |
| 10 | 96.1 (3.76) | 96.5 (3.42) |
| 15 | 95.7 (3.73) | 96.4 (3.25) |
| 20 | 96.0 (2.81) | 97.0 (1.76) |
| 25 | 95.8 (2.67) | 97.0 (1.43) |

Le seuil $\lambda$ a été fixé à 0,6 afin de réduire le temps de calcul. La première observation est que tous ces scores semblent assez similaires : tous les scores moyens sont compris entre 95% et 97%, et cela même lorsque uniquement 5 signaux de référence sont utilisés. Tous les écarts types sont plus petits que 5%, ce qui suggère une grande stabilité de la méthode.

Si le nombre de signaux de référence ne semble pas affecter de façon significative les performances moyennes, il influence principalement la robustesse de la méthode. On peut observer que plus le nombre de signaux de référence augmente, plus les écarts-types diminuent, et donc plus la méthode est robuste. Cette constatation fait sens car il est intuitif que plus on a de signaux de référence, plus on a de chance que toutes les pathologies et types de pas soient considérés, ce qui a tendance à uniformiser les résultats pour tous les signaux de la base de test. Lorsque |P| = 5, on observe une dissymétrie entre les écarts-type de la précision et du rappel. Dû au faible nombre de signaux de référence, on force la correspondance entre des pas pathologiques de petite taille et des pas sains de plus grande taille, ce qui peut provoquer une sur-détection et qui a tendance à faire chuter la précision.

Le tableau 2 ci-dessous présente les précisions et rappels (moyenne, et écart-type entre parenthèses sur 100 simulations) pour différentes valeurs du seuil prédéterminé λ.

Tableau 2

| Valeur de λ | Précision | Rappel |
|---|---|---|
| 0,60 | 95,23 (4,50) | 97,63 (1,61) |
| 0,65 | 93,97 (7,52) | 96,74 (4,58) |
| 0,70 | 95,96 (4,96) | 97,33 (3,22) |
| 0,75 | 96,44 (4,32) | 97,19 (1,99) |
| 0,80 | 97,03 (1,02) | 96,59 (1,89) |
| 0,85 | 97,83 (2,92) | 95,26 (1,93) |
| 0,90 | 98,49 (4,74) | 90,36 (2,51) |

Lorsque le seuil prédéterminé λ augmente, seuls les signaux de référence qui sont très corrélées aux modèles sont sélectionnés: cela augmente la précision. Au contraire, lorsque le seuil prédéterminé λ diminue le rappel augmente et la précision diminue. Ces résultats confirment l'utilité du paramètre λ : λ en augmentant à une valeur appropriée (de l'ordre de 0,6 à 0,8), il est possible d'augmenter la précision (et à la robustesse de la précision) tout en conservant la constante de rappel. En outre, λ a une incidence sur le coût de calcul. Par exemple, en utilisant λ = 0,8 au lieu de λ = 0 (équivalent à l'algorithme sans λ) il est possible de calculer le résultat d'environ 2 fois plus rapidement. Il est donc intéressant d'utiliser la plus grande valeur de λ possible. Les meilleures performances sont obtenues pour λ = 0,8, ce qui constitue un bon compromis entre le rappel et la précision.

Le tableau 3 ci-dessous présente les précisions et rappels (moyenne, et écart-type entre parenthèses sur 100 simulations) pour différentes valeurs du seuil prédéterminé μ.

Tableau 3

| Valeur de μ | Précision | Rappel |
|---|---|---|
| 0,05 | 96,23 (2,29) | 97,42 (0,93) |
| 0,10 | 95,92 (5,66) | 97,04 (1,60) |
| 0,15 | 97,03 (4,02) | 96,59 (1,89) |
| 0,20 | 96,72 (6,30) | 95,78 (1,89) |
| 0,25 | 97,68 (2,67) | 95,25 (1,31) |
| 0,30 | 97,84 (4,94) | 93,88 (2,73) |

Le paramètre μ influence principalement le rappel. En effet, lorsque μ est trop grande, toutes les mesures dont l'amplitude est trop différente de celle des signaux de référence ne sont pas considérées. Cela a un double effet : si l'un des signaux de référence correspond à un patient pathologique dont les étapes ont une faible amplitude, alors il ne sera pas en mesure de détecter des mesures sur des patients en bonne santé. La situation inverse peut également se produire. En effet, lorsque μ augmente, l'effet de normalisation fournie par le coefficient de corrélation de Pearson est neutralisé. Le tableau 3 montre que, de préférence, μ ne devrait pas être supérieure à 0,2 de sorte que le rappel ne soit pas trop faible.

Cette étape, basée sur 100 simulations, a conduit à tester 21000 configurations différentes. Parmi ces configurations, il est possible de mentionner la configuration : |P| = 5 signaux de référence, λ = 0,75 et μ = 0,1, avec un rappel moyen de 98,40% and une précision moyenne de 98,44%. Ce choix de paramètre est un exemple et les paramètres pourront être modifiés en fonction du contenu de la base de données de pas et des séquences à analyser.

Les bonnes performances de la méthode selon l'invention sont liées aux signaux de références qui composent la bibliothèque. Il est remarquable que cette méthode ne nécessite qu'un petit nombre de signaux de références, ce qui tend à montrer que l'algorithme n'a pas besoin d'une grande bibliothèque pour effectuer avec précision. Par exemple, la méthode optimisée comprend 5 signaux de références : 1 signal de référence appartenant à un sujet sain, 3 signaux de références correspondant à des maladies neurologiques, et 1 signal de référence associé aux maladies orthopédiques.

La méthode selon l'invention présente en outre l'avantage d'être capable de détecter avec précision les limites de pas et de s'adapter à différentes durées de pas. Pour 90% des étapes, les erreurs de départ, d'arrivée et la durée de fois sont inférieurs à 0,25 secondes (en valeur absolue). Ces résultats sont satisfaisants par rapport aux annotations incertitudes des experts et des spécialistes.

Le procédé de détection selon l'invention a été comparé avec un procédé de prétraitement de Pan-Tomkins cité en introduction, configuré avec les paramètres suggérés dans l'article « "Automatic step détection in the accelerometer signal,", par H. Ying, C. Silex et al.

Les résultats fournis par ce procédé antérieur sont de faible précision (de l'ordre de 25.6% à 51,20% en fonction des groupes de séquences utilisés). En outre, cette méthode ne permet pas de détecter un instant caractéristique d'un pas tel que pourrait le faire la présente invention (e.g. instant de début de pas ou instant de fin de pas).

La méthode Pan-Tomkins a été optimisée sur l'ensemble de la base de données via le choix de valeurs optimales pour plusieurs paramètres (e.g. fmin, fmax, fenêtre d'intégration, la taille du quartier et le niveau de bruit) de façon à maximiser la F-mesure. Quand optimisé, l'algorithme donne 97,82% de rappel et une précision de 95,72%. Les résultats détaillés sont présentés dans le tableau 4. Alors que ces scores sont comparables à la méthode selon l'invention sur des sujets sains, il est à noter que la méthode Pan-Tomkins a de la difficulté à traiter des sujets présentant des troubles tels que des troubles neurologiques ou orthopédiques. En particulier, sur ces sujets, une sur-détection se produit, réduisant ainsi la précision. Cela montre une limite de méthodes de détection de pas classiques basées sur le traitement du signal: si les signaux à étudier ont des propriétés différentes (bruit, contenu fréquentiel, amplitudes), il est difficile de trouver un traitement unique et adapté à tous les signaux.

Tableau 4

| | Invention | | Pan-Tomkins | |
|---|---|---|---|---|
| | Rappel | Précision | Rappel | Précision |
| Sujets Sains | 98,93 (2,22) | 98,98 (2,43) | 99,14 (1,71) | 97,09 (3,78) |
| Sujets avec troubles orthopédiques | 97,54 (2,92) | 98,77 (2,12) | 98,78 (2,09) | 94,87 (5,09) |
| Sujets avec troubles orthopédiques | 98,55 (3,05) | 98,05 (3,02) | 96,80 (3,52) | 95,49 (4,55) |
| **Total** | 98,40 (2,89) | 98,44 (2,72) | 97,82 (3,07) | 95,72 (4,56) |

En définitive, le procédé de détection mis en oeuvre conformément à l'invention obtient de robustes performances, même en utilisant un petit nombre de signaux de référence.

Le procédé de détection selon l'invention peut trouver de multiples applications. Le procédé de détection peut en particulier servir de phase préliminaire au diagnostic ou au pronostic de trouble de la démarche. Toutefois aucun procédé de diagnostic n'est revendiqué. Etant donné qu'il utilise des données ayant été préalablement mesurées, il ne nécessite pas d'interaction avec le sujet cible pour être mis en oeuvre.

**Revendications**

1. Procédé de caractérisation de démarche comprenant des étapes de :

   • réception d'un signal de mesure de mouvement d'un sujet cible préalablement mesuré,
   • comparaison (204) d'au moins une portion du signal de mesure avec une pluralité de signaux de référence, la pluralité de signaux de référence comprenant au moins un signal de mouvement préalablement mesuré sur un sujet de référence différent du sujet cible, chaque comparaison du signal de mesure avec un signal de référence produisant un coefficient de similarité représentatif d'une probabilité de similarité entre les signaux comparés, dans lequel un premier coefficient de similarité formant un premier maximum local est produit par comparaison d'une première portion du signal de mesure avec un signal de référence, et un deuxième coefficient de similarité formant un deuxième maximum local produit par comparaison d'une deuxième portion du signal de mesure avec le même signal de référence que le premier coefficient de similarité, la deuxième portion étant différente de la première portion, sélection (208) d'au moins un maximum local d'intérêt parmi les coefficients de similarité produits, dans lequel :

o un maximum local parmi les coefficients de similarités produits n'est pas sélectionné comme maximum local d'intérêt lorsque sa valeur est inférieure à un seuil prédéterminé ($\lambda$), de sorte à éliminer des maxima locaux ayant des valeurs de probabilité de similarité faibles,

o le premier maximum local est sélectionné, puis le deuxième maximum local n'est pas sélectionné lorsque la deuxième portion chevauche temporellement la première portion,

dans lequel la pluralité de signaux de référence comprend :

• au moins un signal de mouvement préalablement mesuré sur un sujet de référence atteint d'un trouble de la démarche, et
• au moins un signal de mouvement préalablement mesuré sur un sujet de référence sain.

2. Procédé selon la revendication précédente, comprenant en outre une étape d'identification (210), dans le signal de mesure, d'au moins un instant de repère caractéristique d'un pas du sujet cible, en fonction du signal de référence à partir duquel le maximum local d'intérêt sélectionné a été produit.

3. Procédé selon la revendication précédente, dans lequel

• chaque signal de référence comprend au moins un instant de repère prédéterminé ($t_a$) et caractéristique d'un pas d'un sujet de référence,
• au moins un signal de référence est comparé avec plusieurs portions du signal de mesure temporellement décalées les unes des autres,
• l'instant de repère caractéristique d'un pas du sujet cible est identifié (210) à partir de l'instant de repère prédéterminé du signal de référence à partir duquel a été produit le maximum local d'intérêt sélectionné.

4. Procédé selon l'une des revendications 2 à 3, comprenant le calcul d'un rapport entre des caractéristiques dimensionnelles des deux signaux comparés, et dans lequel l'étape d'identification (210) n'est pas mise en oeuvre pour un maximum local produit à partir des signaux comparés dont le rapport de dimension calculé franchit un seuil prédéterminé.

5. Procédé selon l'une des revendications précédentes, dans lequel :

• au moins un signal de référence est comparé (204) avec plusieurs portions du signal de mesure temporellement décalées les unes des autres, de sorte à produire une séquence de coefficients de similarité ordonnée d'après les positions temporelles des portions du signal de mesure,
• un coefficient de similarité d'une séquence est sélectionné comme maximum local (206) en fonction du résultat d'une comparaison entre la valeur du coefficient avec la valeur du coefficient qui le précède dans la séquence, et du résultat d'une comparaison entre la valeur du coefficient avec la valeur du coefficient qui le suit dans la même séquence.

6. Procédé selon l'une des revendications précédentes, dans lequel le signal de mesure est représentatif du mouvement d'un pied du sujet cible.

7. Système de caractérisation de démarche, comprenant :

• une interface de réception configurée pour recevoir un signal de mesure de mouvement d'un sujet cible, et
• au moins un processeur configuré pour :

o comparer le signal de mesure avec une pluralité de signaux de référence, la pluralité de signaux de référence comprenant au moins un signal de mouvement préalablement mesuré sur un sujet de référence différent du sujet cible, chaque comparaison du signal de mesure avec un signal de référence produisant un coefficient de similarité représentatif d'une probabilité de similarité entre les signaux comparés, dans lequel un premier coefficient de similarité formant un premier maximum local est produit à partir d'une première portion du signal de mesure, et un deuxième coefficient de similarité formant un deuxième maximum local est produit à partir d'une deuxième portion du signal de mesure différente de la première portion,

◦ sélectionner au moins un maximum local parmi les coefficients de similarité produits, dans lequel un maximum local parmi les coefficients de similarités produits n'est pas sélectionné comme maximum local d'intérêt lorsque sa valeur est inférieure à un seuil prédéterminé ($\lambda$), de sorte à éliminer des maxima locaux ayant des valeurs de probabilité de similarité faibles, et dans lequel le premier maximum local est sélectionné, puis le deuxième maximum local n'est pas sélectionné lorsque la deuxième portion chevauche temporellement la première portion,

dans lequel la pluralité de signaux de référence comprend au moins un signal de mouvement préalablement mesuré sur un sujet de référence atteint d'un trouble de la démarche, et au moins un signal de mouvement préalablement mesuré sur un sujet de référence sain.

8. Système selon la revendication précédente, dans lequel le processeur est configuré pour compter le nombre de maximum local d'intérêt sélectionné correspondant à un signal de référence donné, chaque signal de référence étant associé à une information caractéristique du sujet de référence correspondant.

**Patentansprüche**

1. Verfahren zur Charakterisierung eines Ganges, umfassend die Schritte:

    • Empfangen eines zuvor gemessenen Messsignals der Bewegung eines Zielsubjekts,
    • Vergleichen (204) mindestens eines Abschnitts des Messsignals mit einer Vielzahl von Referenzsignalen, wobei die Vielzahl von Referenzsignalen mindestens ein Bewegungssignal umfasst, das zuvor an einem Referenzsubjekt gemessen wurde, das sich vom Zielsubjekt unterscheidet, wobei jeder Vergleich des Messsignals mit einem Referenzsignal einen Ähnlichkeitskoeffizienten erzeugt, der für eine Wahrscheinlichkeit der Ähnlichkeit zwischen den verglichenen Signalen repräsentativ ist, wobei ein erster Ähnlichkeitskoeffizient, der ein erstes lokales Maximum bildet, durch Vergleichen eines ersten Abschnitts des Messsignals mit einem Referenzsignal erzeugt wird, und ein zweiter Ähnlichkeitskoeffizient, der ein zweites lokales Maximum bildet, durch Vergleichen eines zweiten Abschnitts des Messsignals mit dem gleichen Referenzsignal wie beim ersten Ähnlichkeitskoeffizient erzeugt wird, wobei der zweite Abschnitt vom ersten Abschnitt unterschiedlich ist, Auswählen (208) mindestens eines lokalen Maximums von Interesse aus den erzeugten Ähnlichkeitskoeffizienten, wobei:

        ◦ ein lokales Maximum aus den erzeugten Ähnlichkeitskoeffizienten nicht als lokales Maximum von Interesse ausgewählt wird, wenn sein Wert kleiner als ein vorbestimmter Schwellenwert ($\lambda$) ist, so dass die lokalen Maxima mit schwachen Werten einer Ähnlichkeitswahrscheinlichkeit entfernt werden
        ◦ das erste lokale Maximum ausgewählt wird, dann das zweite lokale Maximum nicht ausgewählt wird, wenn der zweite Abschnitt den ersten Abschnitt zeitweilig überlagert,

    wobei die Vielzahl von Referenzsignalen umfasst:

        • mindestens ein Bewegungssignal, das zuvor bei einem Referenzsubjekt mit einer Gangstörung gemessen wurde, und
        • mindestens ein Bewegungssignal, das zuvor bei einem gesunden Referenzsubjekt gemessen wurde.

2. Verfahren nach vorhergehendem Anspruch, umfassend ferner einen Identifikationsschritt (210) im Messsignal mindestens eines Bezugszeitpunkts, der für einen Schritt des Zielsubjekts charakteristisch ist, in Abhängigkeit vom Referenzsignal, von dem ausgehend das ausgewählte lokale Maximum von Interesse erzeugt wurde.

3. Verfahren nach vorhergehendem Anspruch, wobei

    • jedes Referenzsignal mindestens einen vorbestimmten und für einen Schritt eines Referenzsubjekts charakteristischen Bezugszeitpunkt ($t_a$) umfasst,
    • mindestens ein Referenzsignal mit mehreren zeitlich zueinander verschobenen Abschnitten des Messsignals verglichen wird,

• der für einen Schritt des Zielsubjekts charakteristische Bezugszeitpunkt ausgehend von dem vorbestimmten Bezugszeitpunkt des Referenzsignals identifiziert wird (210), von dem ausgehend das ausgewählte lokale Maximum von Interesse erzeugt wurde.

4. Verfahren nach einem der Ansprüche 2 bis 3, umfassend das Berechnen eines Verhältnisses zwischen Größenmerkmalen der beiden verglichenen Signale, und wobei der Identifikationsschritt (210) für ein ausgehend von den verglichenen Signalen erzeugtes lokales Maximum nicht durchgeführt wird, dessen berechnetes Größenverhältnis einen vorbestimmten Schwellenwert überschreitet.

5. Verfahren nach einem der vorhergehenden Ansprüche, wobei:

• mindestens ein Referenzsignal mit mehreren zeitlich zueinander versetzten Abschnitten des Messsignals verglichen wird (204), um eine nach den zeitlichen Positionen der Abschnitte des Messsignals geordnete Folge von Ähnlichkeitskoeffizienten zu erzeugen,
• ein Ähnlichkeitskoeffizient einer Sequenz als lokales Maximum (206) in Abhängigkeit von dem Ergebnis eines Vergleichs zwischen dem Wert des Koeffizienten mit dem Wert des Koeffizienten, der ihm in der Sequenz vorausgeht, und dem Ergebnis eines Vergleichs zwischen dem Wert des Koeffizienten mit dem Wert des Koeffizienten, der ihm in derselben Sequenz folgt, ausgewählt wird.

6. Verfahren nach einem der vorhergehenden Ansprüche, wobei das Messsignal für die Bewegung eines Fußes des Zielobjekts repräsentativ ist.

7. System zur Charakterisierung des Ganges, umfassend:

• eine Empfangsschnittstelle, die ausgelegt ist, um ein Messsignal der Bewegung eines Zielsubjekts zu empfangen, und
• mindestens einen Prozessor, der ausgelegt ist, um:

◦ das Messsignal mit einer Vielzahl von Referenzsignalen zu vergleichen, wobei die Vielzahl von Referenzsignalen mindestens ein Bewegungssignal umfasst, das zuvor bei einem Referenzsubjekt gemessen wurde, das sich vom Zielsubjekt unterscheidet, wobei jeder Vergleich des Messsignals mit einem Referenzsignal einen Ähnlichkeitskoeffizienten erzeugt, der für eine Wahrscheinlichkeit der Ähnlichkeit zwischen den verglichenen Signalen repräsentativ ist, wobei ein erster Ähnlichkeitskoeffizient, der ein erstes lokales Maximum bildet, ausgehend von einem ersten Abschnitt des Messsignals erzeugt wird, und ein zweiter Ähnlichkeitskoeffizient, der ein zweites lokales Maximum bildet, ausgehend von einem zweiten Abschnitt des Messsignals erzeugt wird, der vom ersten Abschnitt unterschiedlich ist,
◦ mindestens ein lokales Maximum aus den erzeugten Ähnlichkeitskoeffizienten auszuwählen, wobei ein lokales Maximum aus den erzeugten Ähnlichkeitskoeffizienten nicht als lokales Maximum von Interesse ausgewählt wird, wenn sein Wert kleiner als ein vorbestimmter Schwellenwert ($\lambda$) ist, so dass die lokalen Maxima mit schwachen Werten einer Ähnlichkeitswahrscheinlichkeit entfernt werden, und wobei das erste lokale Maximum ausgewählt wird, dann das zweite lokale Maximum nicht ausgewählt wird, wenn der zweite Abschnitt den ersten Abschnitt zeitweilig überlagert,

wobei die Vielzahl von Referenzsignalen mindestens ein Bewegungssignal umfasst, das zuvor bei einem Referenzsubjekt mit einer Gangstörung gemessen wurde, und mindestens ein Bewegungssignal, das zuvor bei einem gesunden Referenzsubjekt gemessen wurde.

8. Verfahren nach vorhergehendem Anspruch, wobei der Prozessor ausgelegt ist, um die Anzahl des ausgewählten lokalen Maximums von Interesse zu zählen, die einem bestimmten Referenzsignal entspricht, wobei jedes Referenzsignal einer Information zugeordnet ist, die für das entsprechende Referenzsubjekt charakteristisch ist.

**Claims**

1. A method of gait characterization comprising the steps of:

• reception of a motion measurement signal from a previously measured target subject,
• comparison (204) of at least a portion of the measurement signal with a plurality of reference signals, the plurality of reference signals comprising at least one motion signal previously measured on a reference subject different from the target subject, each comparison of the measurement signal with a reference signal producing a similarity coefficient representative of a probability of similarity between the compared signals, in which a first similarity coefficient forming a first local maximum is produced by comparing a first portion of the measurement signal with a reference signal, and a second similarity coefficient forming a second local maximum is produced by comparing a second portion of the measurement signal with the same reference signal as the first similarity coefficient, the second portion being different from the first portion, selecting (208) at least one local maximum of interest from the similarity coefficients produced, in which :

    ◦ a local maximum among the produced similarity coefficients is not selected as the local maximum of interest when its value is below a predetermined threshold ($\lambda$), so as to eliminate local maxima with low similarity probability values,
    ◦ the first local maximum is selected, then the second local maximum is not selected when the second portion temporally overlaps the first portion,

wherein the plurality of reference signals comprises :

    • at least one movement signal previously measured on a reference subject suffering from a gait disorder, and
    • at least one motion signal previously measured on a healthy reference subject.

2. A method according to the preceding claim, further comprising a step of identifying (210), in the measurement signal, at least one reference time characteristic of a step of the target subject, as a function of the reference signal from which the selected local maximum of interest was produced.

3. Process according to the preceding claim, in which

    • each reference signal comprises at least one predetermined reference time ($t_a$) characteristic of a reference subject step,
    • at least one reference signal is compared with several portions of the measurement signal that are temporally offset from one another,
    • the landmark time characteristic of a target subject step is identified (210) from the predetermined landmark time of the reference signal from which the selected local maximum of interest was generated.

4. A method according to one of claims 2 to 3, comprising calculating a ratio between dimensional characteristics of the two compared signals, and wherein the identification step (210) is not implemented for a local maximum produced from the compared signals whose calculated dimension ratio crosses a predetermined threshold.

5. Process according to one of the preceding claims, in which :

    • at least one reference signal is compared (204) with several portions of the measurement signal temporally offset from one another, so as to produce a sequence of similarity coefficients ordered according to the temporal positions of the portions of the measurement signal,
    • a similarity coefficient of a sequence is selected as a local maximum (206) according to the result of a comparison between the value of the coefficient with the value of the coefficient that precedes it in the sequence, and the result of a comparison between the value of the coefficient with the value of the coefficient that follows it in the same sequence.

6. Method according to one of the preceding claims, wherein the measurement signal is representative of the movement of a foot of the target subject.

7. Gait characterization system, comprising :

    • a receiving interface configured to receive a motion measurement signal from a target subject, and
    • at least one processor configured to :

        o comparing the measurement signal with a plurality of reference signals, the plurality of reference signals comprising at least one motion signal previously measured on a reference subject different from the target subject, each comparison of the measurement signal with a reference signal producing a similarity coefficient representative of a probability of similarity between the compared signals, wherein a first similarity coefficient forming a first local maximum is produced from a first portion of the measurement signal, and a second similarity coefficient forming a second local maximum is produced from a second portion of the measurement signal different from the first portion,

○ select at least one local maximum from the generated similarity coefficients, wherein the first local maximum is selected and then the second local maximum is not selected when the second portion temporally overlaps the first portion,

wherein the plurality of reference signals comprises at least one movement signal previously measured on a reference subject suffering from a gait disorder, and at least one movement signal previously measured on a healthy reference subject.

8. A system according to the preceding claim, in which the processor is configured to count the number of local maximums of selected interest corresponding to a given reference signal, each reference signal being associated with information characteristic of the corresponding reference subject.

FIG. 1

FIG. 2

| | |
|---|---|
| Acquisition de signaux témoins | 102 |
| Mémorisation signaux témoins | 104 |
| Identification d'instant(s) caractéristique(s) dans les signaux témoins | 106 |

FIG. 3a

Accélération verticale (m/s²)
Accélération axe Z (m/s²)
Vitesse angulaire axe Y (rad/s)

Accélération verticale (m/s²)
Accélération axe Z (m/s²)
Vitesse angulaire axe Y (rad/s)

FIG. 3b

```
┌──────────────────────────────────────┐  ⌐ 202
│  Acquisition d'un signal cible sur un sujet cible │
└──────────────────────────────────────┘
                      │
                      ▼
┌──────────────────────────────────────┐  ⌐ 204
│  Comparaison entre une portion du signal cible │
│           et un signal témoin          │
└──────────────────────────────────────┘
             Coefficients │ de similarité
                      ▼
┌──────────────────────────────────────┐  ⌐ 206
│  Identification de maxima locaux parmi les │
│         coefficients de similarités     │
└──────────────────────────────────────┘
               Maxima │ locaux
                      ▼
┌──────────────────────────────────────┐  ⌐ 208
│     Sélection de maxima locaux d'intérêt  │
└──────────────────────────────────────┘
              Maxima │ locaux d'intérêt
                      ▼
┌──────────────────────────────────────┐  ⌐ 210
│  Identification d'instant(s) de repère dans le │
│            signal de référence          │
└──────────────────────────────────────┘

┌──────────────────────────────────────┐  ⌐ 212
│  Comptage des signaux témoins ayant produit │
│        des maxima locaux d'intéret      │
└──────────────────────────────────────┘
```

FIG. 4

FIG. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2439492 A1, HONEYWELL

- US 2007021689 A1, STERGIOU NIKOLAOS

**Littérature non-brevet citée dans la description**

- **J. PAN** ; **W. J TOMPKINS**. *A real-time QRS détection algorithm*

- **H. YING** ; **C. SILEX**. *Automatic step détection in the accelerometer signal*